Europäisches Patentamt

European Patent Office

Office européen des brevets

(11) Publication number: **0 113 183**
**A1**

(12) **EUROPEAN PATENT APPLICATION**

(21) Application number: **83307227.5**

(22) Date of filing: **28.11.83**

(51) Int. Cl.³: **C 12 N 1/38**
**C 12 G 3/02**

(30) Priority: **01.12.82 GB 8234279**
**16.05.83 GB 8313428**
**21.09.83 GB 8325314**

(43) Date of publication of application:
**11.07.84 Bulletin 84/28**

(84) Designated Contracting States:
**AT BE CH DE FR GB IT LI NL SE**

(71) Applicant: **Carson, Thomas Anthony**
**Red Lodge Highfields**
**Eye Suffolk(GB)**

(72) Inventor: **Carson, Thomas Anthony**
**Red Lodge Highfields**
**Eye Suffolk(GB)**

(74) Representative: **Dummett, Thomas Ian Peter**
**7 Hasketon Road**
**Woodbridge Suffolk IP12 4JT(GB)**

(54) Fermentation method and composition for use therein.

(57) A method for fermenting an aqueous sugar solution which method comprises maintaining the pH of the fermenting mixture within the range 3 to 8 at substantially all times during the fermentation of the available sugar by the presence in the fermenting mixture of a material which is substantially insoluble in the fermenting mixture and which comprises or releases a substance which neutralises acidic materials produced during the fermentation process so as to control the pH of the fermenting mixture. Preferably, the pH controlling material is calcium carbonate.

A composition for use in the method of the invention and a unitary pack comprising ingredients in specified proportions is also described.

Croydon Printing Company Ltd.

TITLE: FERMENTATION METHOD AND COMPOSITION FOR USE THEREIN

The present invention relates to a method for improving the fermentation of sugars, notably to produce substantially flavourless and colourless alcohol bases for use in making mixer drinks; and to a composition for use therein.

Fruit and vegetable mashes have been fermented to give wines, beers and other beverages for many years. It has been proposed to include a water-soluble buffering agent in the fermenting mixture to prevent the pH from falling to a value below about 3.5. Typical of such buffering agents is potassium phosphate. However, fermentation of such mixtures often does not go to completion, or fermentation occurs only slowly. No satisfactory solution to the slowing down or stopping of the fermentation has been found. The partially fermented product contains a low ethanol content and unfermented sugar which is wasteful and has an unacceptably sweet flavour. Also, problems are encountered with such incompletely fermented products if a secondary fermentation is to be carried out to give a sparkling drink.

These problems are a particular detriment when it is desired to produce a substantially colour and flavour free alcohol base which can be flavoured to simulate gin and tonic or other spirit based beverages as described in my co-pending Application No. 82/30318.

TIP/81/2                    -2-

I have found that the fermentation stops or slows down due to an excessive drop in pH during the fermentation. This occurs particularly where substantially pure sugars provide substantially all of the carbohydrate being fermented. It has been proposed in for example BP 801612 to use a fermentation aid containing peptone and vitamins to reduce the problems of stopped fermentation. However, the peptone introduces off-flavours into the product. In an attempt to overcome these problems, I have added larger than usual amounts of the conventional buffering agents. However, this also results in a product with a totally unacceptable taste, akin to brine.

In my co-pending application no 82/34729 I have described a fermentation aid which contains amino acids and vitamins and these enable improved fermentation to be achieved. Calcium salts are also said to be present and I have found that some of these can react with the acids released during fermentation. They can thus be used provide an important buffering effect even when substantially pure sugars are used. Since calcium salts are often dissolved to only a small extent in the fermenting mixture during the fermentation period, salts can be selected which do not find their way into the final product to impart flavour thereto. Other forms of slightly soluble acid neutralising materials can also be used in place of the calcium salts.

TIP/81/3                          -3-

Accordingly, the present invention provides a method for fermenting an aqueous sugar solution using a yeast, which method comprises maintaining the pH of the fermenting mixture within the range 3 to 8, preferably 3.5 to 7 notably 4 to 6, at substantially all times during the fermentation of the available sugar by the presence in the fermenting mixture of a material which is substantially insoluble in the fermenting mixture and which comprises or releases a substance which neutralises acidic materials produced during the fermentation process so as to control the pH of the fermenting mixture.

The term acidic material is used herein and in the claims to denote in general terms materials which lower the pH of the fermenting mixture and includes free acids and partial salts thereof.

The invention also provides a fermentation aid composition, preferably as a dry mix of the ingredients, which comprises a material which is substantially insoluble in the mixture of products obtained during the fermentation of a sugar, which material comprises or releases a substance which is physiologically compatible with the components of the fermentation mixture and neutralises acidic materials produced during the fermentation of a sugar so as to control the pH of the fermenting mixture; in association with one or more other ingredients to be incorporated into the fermentation mixture to aid fermentation of the sugar, eg. sugar, yeast, inorganic sources of nitrogen and/or potassium and/or phosphorus.

The pH controlling material for present use can be one which slowly releases a base or alkali into the fermenting mixture. Preferably, the material is one which releases its active component over 72 to 110 hours at 25° C in a 6% by volume alcohol solution. The material can, for example, be only slowly soluble in the mixture, or can be contained in a protective coating or matrix which is slowly dissolved or broken down by the fermenting mixture. Thus, for example, a water-soluble alkali or base, such as sodium or potassium hydroxide or carbonate, can be encapsulated in a water and/or alcohol-soluble or acid-decomposable polymer coating. Such coated particles can be made by a number of methods, eg. using the solvent precipitation methods developed by the National Cash Register Company. Alternatively, such a base or alkali can be dispersed in a polymer matrix having a porous structure from which the base or alkali is leached slowly due to the capillary size of the pores in the polymer matrix. Thus, for example a porous PVA polymeric block can be impregnated, eg. under vacuum, with a solution of a base to form a slow release material with acid neutralising properties. Alternatively, the alkali can be contained within a glass or other frit which is slowly dissolved to release the alkali.

A particularly preferred form of pH controlling material for present use is one which is substantially insoluble in the fermenting mixture and which reacts with the acidic material as it is formed during the fermentation. Such a material can be in the form of a complex which is substantially inert in the fermentation mixture, but which is decomposed by the acidic material to release an alkali or base.

Alternatively, the pH controlling material can be provided by a base or alkali which remains substantially undissolved in the fermenting mixture until acidic material is released. It then reacts with the acidic material substantially as that is formed. Preferably, the alkali or base forms a substantially insoluble salt of the acidic material which can be separated off by decanting or by filtration from the fermentation products. Thus, a particularly suitable pH controlling material for present use is a carbonate or bi-carbonate of calcium and/or magnesium.

From a particularly preferred aspect, the present invention therefore provides a method for fermenting an aqueous sugar solution which comprises fermenting the sugar in the presence of a substantially water-insoluble calcium and/or magnesium salt, preferably of an acid which is weaker than those acids formed during fermentation of the sugar, present in an amount sufficient to maintain the pH of the fermenting mixture within the range 3 to 8.

The invention can be applied to the fermentation of a wide range of types of sugar, eg. those derived from fruits such as apple, grape, blackberry and other soft fruits; from vegetable mashes, such as sugar cane, beet or grain mashes; and honey. However, the method of the invention is of especial use with sugars which are low in proteinaceous material, notably those which have less than 10% w/w of the total fermentable carbohydrate provided in the form of fruit or vegetable mashes, juices or concentrates. Thus, the invention can be applied to the fermentation of substantially pure sucrose, glucose, fructose, galactose, maltose, lactose or other mono- or di-saccharide solutions, including modified sugars such as invert sugar. The term sugar is therefore used herein in a general sense to denote any saccharide wherever the context permits .

Fermentation of the sugar solution is carried out in the conventional manner by mixing a yeast, the pH controlling material and a fermentation aid and/or nutrient with the sugar solution and allowing the mixture to ferment out, eg. at a temperature of from 40 to 95°F. The fermentation aid is preferably of the type described in my co-pending application no. 8234279 and contains at least one vitamin from the B complex and essential protein amino acids.

Preferably, the vitamins for present use include $B_6$ and may include vitamins from other groups. Especially preferred vitamins for present use are selected from the group consisting of p-amino benzoic acid, biotin, folic acid, inositol, nicotinic acid, pantothenic acid, pyridoxine, riboflavin and thiamine. It is preferred to use a mixture of two or more B vitamins in the fermenting mixture. Typically, each of the B vitamins will be used in an amount of from 0.02 to 1000, preferably 1 to 100, millegrams per litre of the mixture being fermented, and the total mixture of vitamins is used in amount of from 0.1 to 5000, preferably 10 to 500, millegrams per litre.

Preferred proteinaceous amino acids are selected from the group consisting of alanine, arginine, aspartic acid, asparagine, cysteine, glutamic acid, glutamine, glycine, histidine, isoleucine, leucine, lysine, methionine, phenylalanine, proline, serine, threonine, tryptophane, tyrosine and valine. It will usually be desired to use a mixture containing 2, 3 or more of the amino acids in the mixture being fermented. The optimum mixture of amino acids selected will depend upon the yeast being used to ferment the carbohydrates and can be readily determined by simple trial and error tests. If desired, the amino acids can be used in the form of a salt thereof, notably as the hydrochloride or the calcium salt. Typically, the amino acids will each be used in an amount of from 0.5 to 250, preferably 1 to 100, millegrams per litre of the mixture being fermented, and the total mixture of amino acids is used in an amount of from 1 to 10000, preferably 5 to 500, millegrams per litre.

The fermentation mixture may also contain other ingredients: eg. inorganic sources of nitrogen and/or phosphorus and/or potassium, for example ammonium sulphate and/or phosphate; and trace metal salts, eg. salts of sodium, boron, copper, manganese or zinc, eg sodium chloride. These other materials are used in the conventional amounts for fermentation, eg. the nitrogen sources will typically supply from 0.1 to 10 gms of N per litre of the mixture being fermented. If desired, flavouring enhancement agents, eg. citric acid, can also be present or can be added after fermentation is carried out.

The pH controlling material substantially replaces the water-soluble buffering agents used conventionally. Preferably, it is added to the initial mixture to be fermented with the other ingredients. Part may be added later if desired, provided that there is present at substantially all times during fermentation sufficient material to react with the acidic materials as they are released.

It is preferred to incorporate an excess of the pH controlling material into the fermenting mixture to ensure that sufficient is present at substantially all times to maintain the pH of the fermenting mixture within the range 3 to 8, notably 4 to 6. The optimum amount can readily be determined for any given case. In the case of chalk, limestone or calcium carbonate BP, it is preferred to use sufficient to provide from 0.5 to 5 gms of 100% calcium carbonate per litre of the liquid phase of the fermentation mixture.

As indicated above, the pH controlling material is preferably put up in association with one or more of the other ingredients for the fermentation mixture. Thus, the present invention also provides a composition comprising a pH controlling material physiologically compatible with the components of a fermenting sugar solution in association with one or more components selected from: a sugar, a vitamin selected from the B group, an essential protein amino acid, a yeast and/or a source of inorganic nitrogen, phosphorus and/or potassium physiologically compatible with the ingredients of a fermenting sugar solution. Preferably, the pH controlling material is selected from calcium and/or magnesium carbonate and/or bi-carbonate and is dry mixed with one or more of the other ingredients or is incorporated

TIP/81/10                           -10-

in an aqueous solution or suspension of one or more of the other ingredients, optionally a preservative, eg. sodium metabisulphite, is also present.

Preferably, the association of ingredients is put up in a kit form for mixture with the required amount of water and sugar to give the fermentation mixture. Typically the ingredients will be put up in substantially sterile containers, eg. plastics bottles or plastics or metal foil sachets, which are in turn put up in an outer container to provide a unitary pack. Typically, such a unitary pack will comprise

a). from 2 to 30 gms of a yeast, preferably a dried yeast;

b). from 25 to 250 gms of a nutrient for the yeast selected from a mixture of substantially pure essential protein amino acids and substantially pure vitamins at least one of which is selected from the B complex, and/or from one or more sources of inorganic nitrogen and/or potassium and/or phosphorus physiologically compatible with a fermentation yeast;

c). from 3 to 60 gms (expressed as active material) of a material which is substantially insoluble in a fermenting sugar mixture and which comprises or releases a substance which neutralises acidic materials produced during the fermentation of a sugar so as to control the pH of the fermenting mixture, preferably calcium and/or magnesium carbonate;

d).     optionally up to 5000 gms of fermentable carbohydrate, which is preferably a substantially pure sugar, optionally containing one or more sythetic sweetening agents, eg. saccharin;

e).     optionally one or more flavouring agents provided other than by the fermentable carbohydrate; and

f).     optionally up to 2000 mls of water as a carrier for one or more of the other ingredients.

As indicated above, the invention is of especial use in the fermentation of substantially pure sugar solutions to produce a substantially taste and colour free alcohol base containing up to 18% by volume ethanol. Such alcohol bases find especial use in the preparation of mixer drinks in the methods of my co-pending applications. Moreover, since the fermentation technique is essentially the same as a conventional home brewing technique, such mixer drinks can be produced in the home.

However, the invention can also be applied to the large scale production of aqueous solutions containing ethanol and other alcohols, eg. fusel oils, from a wide variety of fruit and vegetable mashes or sugar concentrates from which the ethanol can be recovered by distillation for industrial use. The method of the invention enables high levels of of ethanol, eg. from 12 to 18% by volume, to be reached in such fermentations. This reduces the amount of energy required to reuqired in the distillation and hence the cost of producing ethanol by this method.

The invention will now be illustrated by the following Examples in which all parts and percentages are by weight unless stated otherwise:

Example 1:

A fermentation mixture was prepared by dissolving 1000 parts of granulated refined cane sugar in water at 80° C and the mixture made up to 4400 volumes by the addition of cold water. The temperature was adjusted to approximately 25°C and a mixture of 3 parts of yeast and 20 parts of a conventional nutrient formulation was added with stirring to the mixture. The initial specific gravity (SG) of the mixture was 1090.

The mixture was split into two halves and to one half was added the conventional amount of 3 parts of potassium hydrogen phosphate. To the other half was added 6 parts of powdered calcium carbonate BP. The two mixtures were allowed to ferment out for four days at a temperature of approximately 25°C. At the end of this time, the mixture containing the calcium carbonate had fermented out to dryness and had a specific gravity of 0.990.

By way of comparison, the fermentation of the other

TIP/81/13                          -13-

mixture had been slow and it had reached a specific gravity of only 1030. Even after a further seven days the mixture had not fermented out to dryness and had a specific gravity of 1010. Attempts to restart fermentation by aeration and adding extra yeast had no success and this sample had to be discarded as containing an excessively large proportion of unfermented sugars.

The sample containing the calcium carbonate was decanted from the solid residue and filtered to give a substantially clear solution with little or no colour and flavour. This solution was filled into bottles together with a gin and tonic flavouring to undergo secondary fermentation and the bottles stoppered. After a further 20 days an effervescent drink was obtained tasting closely akin to gin and tonic and containing approximately 12% by volume alcohol.

Comparative Example:

The process of Example 1 was repeated using potassium phosphate as the buffering agent at levels of 1, 2, 5 and 10 parts per 1000 parts by volume of the mixture being fermented. In all cases, fermentation ceased or slowed down after 2 days, giving products with SG.s in the range 1010 to 1050 and containing a large proportion of the original carbohydrates unfermented. Once a level of between 5 and 10 parts per 1000 of the buffering agent is reached, the

0113183

product has a noticeable brine flavour.  Increasing the level of buffering agent beyond 10 parts may improve the extent of fermentation but the product had an increasingly accentuated brine flavour.

TIP/81/15                    -15-

CLAIMS:

1). A method for fermenting an aqueous sugar solution which method comprises maintaining the pH of the fermenting mixture within the range 3 to 8 at substantially all times during the fermentation of the available sugar by the presence in the fermenting mixture of a material which is substantially insoluble in the fermenting mixture and which comprises or releases a substance which neutralises acidic materials produced during the fermentation process so as to control the pH of the fermenting mixture.

2). A fermentation aid composition suitable for use in the method of claim 1, which comprises a material which is substantially insoluble in the mixture of products obtained during the fermentation of a sugar, which material comprises or releases a substance which is physiologically compatible with the components of the fermentation mixture and neutralises acidic materials produced during the fermentation of a sugar so as to control the pH of the fermenting mixture; in association with one or more other ingredients to be incorporated into the fermentation mixture to aid fermentation of the sugar.

3). A method or composition as claimed in either of claims 1 or 2 wherein the pH controlling material is selected from one which releases its active component over a period of from 72 to 110 hours at 25° C in a 6% by volume alcohol solution.

4). A method or composition as claimed in any one of the preceding claims wherein the pH controlling material is provided by a base or alkali which remains substantially undissolved in the fermentation mixture and forms a substantially insoluble salt with the acidic material as that is released during fermentation.

5). A method or composition as claimed in any one of the preceding claims wherein the pH controlling material is a carbonate or bi-carbonate of calcium and/or magnesium.

6). A method for fermenting an aqueous sugar solution which comprises fermenting the sugar in the presence of a substantially water-insoluble calcium and/or magnesium salt present in an amount sufficient to maintain the pH of the fermenting mixture within the range 3 to 8.

7). A method as claimed in any one of the preceding claims wherein the pH controlling material comprises calcium carbonate and provides from 0.5 to 5 gms of $CaCO_3$ per litre of liquid phase to be fermented.

8). A method as claimed in any one of the preceding claims wherein the carbohydrate to be fermented has less than 10% w/w thereof provided in the form of a fruit or vegetable mashe, juice or concentrate.

9). A method as claimed in any of the preceding claims wherein a fermentation aid comprising at least one vitamin from the B complex and essential protein amino acids is present in the mixture being fermented.

10). A unitary pack containing ingredients for use in the method of claim 1 which comprises:

a). from 2 to 30 gms of a yeast;

b). from 25 to 250 gms of a nutrient for the yeast selected from a mixture of substantially pure essential protein amino acids and substantially pure vitamins at least one of which is selected from the B complex, and/or from one or more sources of inorganic nitrogen and/or potassium and/or phosphorus physiologically compatible with a fermentation yeast;

c). from 3 to 60 gms (expressed as active material) of a material which is substantially insoluble in a fermenting sugar mixture and which comprises or releases a substance which neutralises acidic materials produced during the fermentation of a sugar so as to control the pH of the fermenting mixture;

d). optionally up to 5000 gms of \fermentable carbohydrate;

e). optionally one or more flavouring agents provided other than by the fermentable carbohydrate; and

f). optionally up to 2000 mls of water as a carrier for one or more of the other ingredients.

ʋ

**0113183**

Application number

# EUROPEAN SEARCH REPORT

EP  83 30 7227

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | EP-A-0 022 341  (STATE OF OREGON) *Claims 1-4,6,7,11,12,14,19,20; page 5, line 32 - page 6, line 5; page 8, lines 31-35; Page 10, lines 1-15; page 10, line 28 - page 11, line 6* | 1-10 | C 12 N   1/38 C 12 G   3/02 |
| X | US-A-4 178 389  (D.P.PILLA) *Claims; example* | 1-10 | |
| X | FR-A- 948 111  (HOFFMANN-LA ROCHE CIE.) *Summary;  example; page 2, lines 6-14* | 1-9 | |

| TECHNICAL FIELDS SEARCHED (Int. Cl. ³) |
|---|
| C 12 G C 12 P C 12 N A 23 C |

The present search report has been drawn up for all claims

| Place of search | Date of completion of the search | Examiner |
|---|---|---|
| THE HAGUE | 21-03-1984 | COUCKE A.O.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO Form 1503. 03.82